# EUROPÄISCHE PATENTANMELDUNG

(11) **EP 0 807 405 A1**
(43) Veröffentlichungstag der Anmeldung: **19.11.1997**
(21) Anmeldenummer: 97107309.3
(22) Anmeldetag: 02.05.1997
(51) Int. Cl.: A61B 6/02

(54) **Verfahren zur Erstellung von Tomosyntheseaufnahmen**

(30) Priorität: 17.05.1996 DE 19619915
(71) Anmelder: SIEMENS AKTIENGESELLSCHAFT, 80333 München (DE)
(72) Erfinder: Plötz, Josef, Dr., 64625 Bensheim (DE)

(57) **Zusammenfassung**

Erfindungsgemäß findet bei der Erstellung einer Tomosyntheseaufnahme eines Untersuchungsobjektes mittels eines Röntgendiagnostikgerätes ein Referenzobjekt (2) Anwendung, das aus wenigstens zwei Teilobjekten (5,6) besteht, die vorzugsweise kreuzweise angeordnet sind. Die Teilobjekte (5,6) können sich auch hinsichtlich der Strahlenabsorption unterscheiden. Das Referenzobjekt hat einen festen Bezug zu Strahlenquelle oder Strahlempfänger, mit der es unmittelbar in Verbindung steht.

## Beschreibung

Aus der WO 93/22 893 A1 ist eine Methode bekannt, mit der es möglich ist, eine Aufnahme eines Untersuchungsobjektes zu rekonstruieren, ohne das hierbei die Projektionswinkel α, die geometrische Anordnung von Strahlensender und Strahlenempfänger und die Fokalebene bekannt sind. Gemäß dieser Methode ist im Bereich des Strahlenempfängers eine Referenz aus strahlenabsorbierendem Material mit bekannter Größe und bekanntem Abstand zum Strahlenempfänger vorgesehen, die bei jeder Einzelprojektion auf den Strahlenempfänger projiziert wird. Aufgrund der örtlichen Abbildung der Referenz auf den Strahlenempfänger für jede Einzelprojektion kann die geometrische Anordnung und der zweidimensionale Projektionswinkel α ermittelt werden.

Eine Halterung zum Positionieren eines Strahlensenders eines Röntgendiagnostikgeräts für Tomosynthese ist aus der DE 44 14 689 A1 bekannt. An die Halterung ist ein Tragarm angekoppelt, an dem - in Strahlungsrichtung gesehen - vor dem Untersuchungsobjekt ein kugelförmiges Referenzobjekt und hinter dem Untersuchungsobjekt ein Strahlenempfänger angeordnet sind. Über die Halterung wird zum einen der Abstand des Strahlensenders zum Referenzobjekt und zum Strahlenempfänger sowie der Winkel α eines vom Strahlensender emittierten Strahlenbündels zu einer Bezugsachse der Haltevorrichtung vorgegeben. Es ist ferner bekannt, die Strahlenquelle verstellbar in einem Gehäuse anzuordnen, an das eine Positionierungsvorrichtung für das Referenzobjekt und den Strahlenempfänger ankoppelbar ist.

Aufgabe der Erfindung ist die weitere vorteilhafte Ausgestaltung eines Verfahrens und einer Vorrichtung zur Erstellung von Tomosyntheseaufnabmen. Insbesondere soll durch eine präzise Erfassung der geometrischen Anordnung von Strahlensender insbesondere Strahlenquelle, Strahlenempfänger und des Untersuchungsobjektes flexiblere und effektivere Möglichkeiten geschaffen werden, die Vorrichtung auszugestalten, das Untersuchungsobjekt zu positionieren und Tomosyntheseaufnahmen aus den Signalen des Strahlenempfängers zu erzeugen.

Dies Aufgabe wird erfindungsgemäß durch ein Verfahren nach dem Patentanspruch 1 und eine Vorrichtung nach dem Patentanspruch 11 gelöst.

Vorteil der Erfindung ist, daß bei dem Verfahren und der Vorrichtung nach der Erfindung ein Referenzobjekt Anwendung findet, das wenigstens aus zwei Teilobjekten besteht. Es kann somit nicht nur der Abstand der Strahlenquelle zum Strahlenempfänger, sondern auch der Einstrahlungswinkel (Projektionswinkel) und die Einstrahlungsrichtung sehr genau bestimmt werden. Auch eine Verdrehung des Strahlenempfängers relativ zum Referenzobjekt kann exakt erfaßt werden. Das Referenzobjekt kann auch unmittelbar mit dem Strahlensender verbunden werden und braucht nicht an den Strahlenempfänger gekoppelt zu sein.

Es ist besonders vorteilhaft, wenn bei dem erfindungsgemäßen Verfahren die Teilobjekte balkenförmig ausgebildet und kreuzförmig angeordnet sind, oder alternativ wenigstens drei Teilobjekte vorgesehen sind, deren Anordnung zueinander von einer Geraden abweicht. Die Teilobjekte können dann besonders vorteilhaft kugel- oder scheibenförmig ausgebildet sein und einen geometrischen Körper bilden. Unterscheiden sich die Teilobjekte hinsichtlich der Strahlenabsorption, so kann somit die Einstrahlungsrichtung besonders präzise bestimmt werden.

Weitere Vorteile und Einzelheiten der Erfindung ergeben sich aus der nachfolgenden Beschreibung eines Ausführungsbeispieles anhand der Zeichnung in Verbindung mit den Unteransprüchen.

Es zeigt:
- FIG 1: ein Röntgendiagnostikgerät für Tomosyntheseaufnahmen in prinzipieller Darstellung, wobei das Referenzobjekt einen festen Bezug zur Strahlensender hat, mit dem es unmittelbar in Verbindung steht,
- FIG 2: ein Röntgendiagnostikgerät für Tomosyntheseaufnahmen in prinzipieller Darstellung, wobei das Referenzobjekt einen festen Bezug zum Strahlenempfänger hat, mit dem es unmittelbar in Verbindung steht,
- FIG 3: ein erstes,
- FIG 4: ein zweites,
- FIG 5: ein drittes und
- FIG 6: ein viertes Ausführungsbeispiel eines Referenzobjektes des Röntgendiagnostikgerätes nach den FIG 1 oder FIG 2.

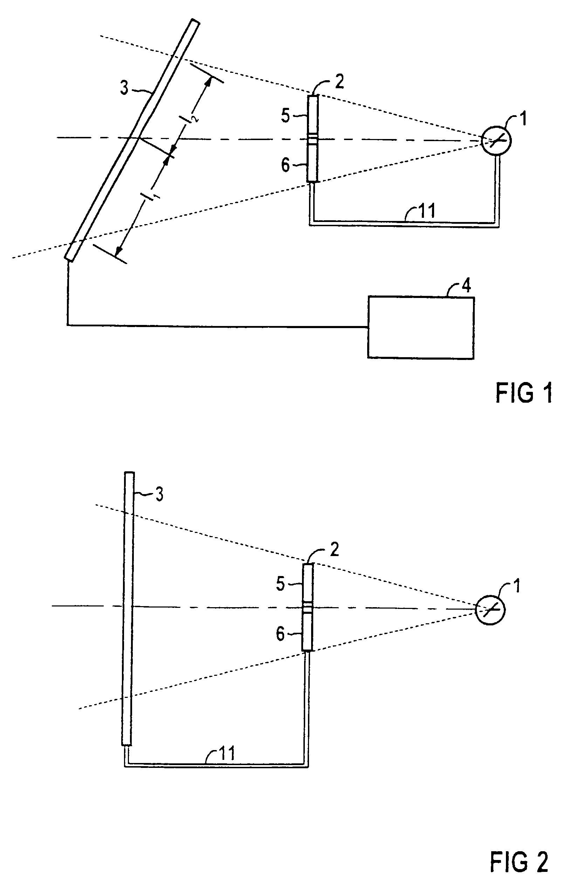

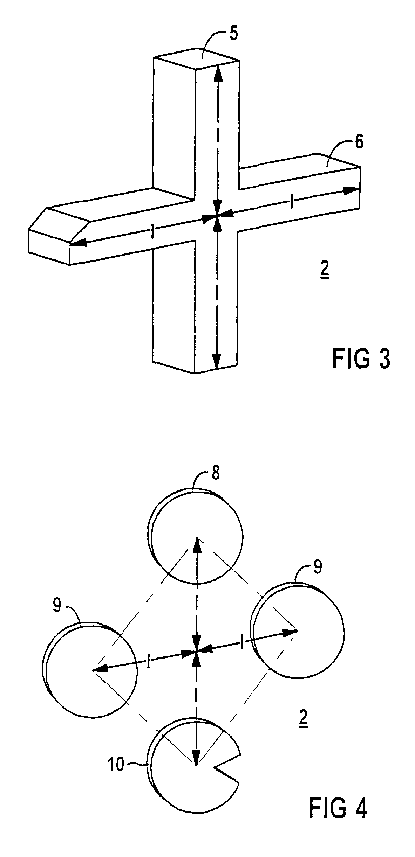

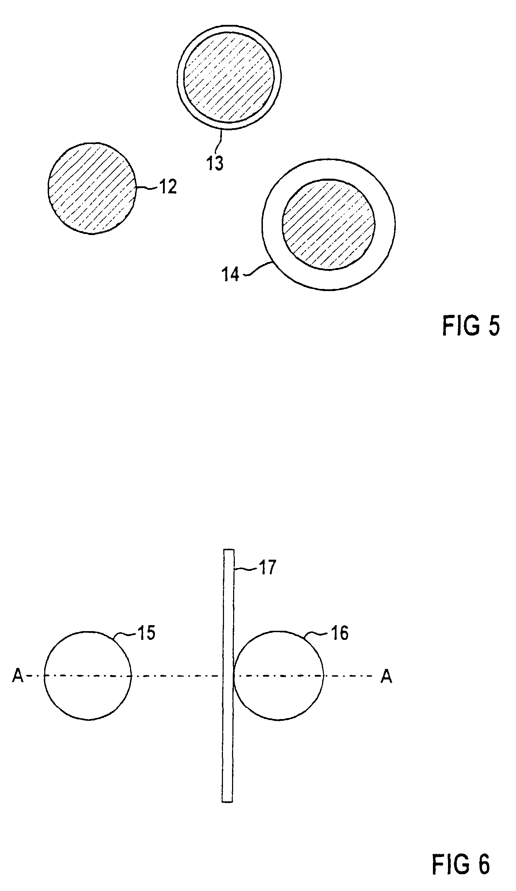

Zum Durchführen des erfindungsgemäßen Verfahrens zur Erstellung einer Tomosyntheseaufnahme eines Untersuchungsobjektes weist ein Röntgendiagnostikgerät einen Strahlensender, insbesondere eine Strahlenquelle 1, ein Referenzobjekt 2 und einen Strahlenempfänger 3 auf. Gemaß der Fig 1 haben hierbei das Referenzobjekt 2 und der Strahlensender, insbesondere die Strahlenquelle 1, beispielsweise über eine Halterung 11, einen festen Bezug zueinander. Die beim Durchstrahlen des Referenzobjektes 2 und des Untersuchungsobjektes vom Strahlenempfänger 3 erzeugten Signale werden einer Bilderzeugungseinrichtung 4 zugeführt, die aus den Signalen des Referenzobjektes 2 den Abstand zum Strahlenempfänger 3 und zum Untersuchungsobjekt sowie den Einstrahlungswinkel (Projektionswinkel) und die Einstrahlungsrichtung und in Verbindung mit den Signalen des Untersuchungsobjektes Bildsignale einer Tomosyntheseaufnahme berechnet. Nach dem erfindungsgemäßen Verfahren zur Erstellung einer Tomosyntheseaufnahme findet ein Referenzobjekt 2 Anwendung, das aus wenigstens zwei Teilobjekten 5,6, besteht, die gemäß einer ersten Variante (FIG 3) balkenförmig ausgebildet sind und sich kreuzen. Nach einer zweiten Variante (FIG 4) findet ein Referenzobjekt 2 Anwendung das wenigstens drei Teilobjekte 7,8,9,10 aufweist, deren Anordnung zueinander von einer Geraden abweicht. Diese Teilobjekte 7,8,9,10 können zum Beispiel kugel-, scheiben-, balken-, stab- oder zylinderförmig ausgebildet sein. Vorzugsweise unterscheidet sich zumindest ein Teilobjekt 6,10, zumindest in einem Teilbereich, hinsichtlich der Strahlenabsorption, von den weiteren Teilobjekten 5,7,8,9, so daß sich die Projektionsrichtungen genau ermitteln lassen, wenn die Abmessungen des Referenzobjektes oder der Teilobjekte 5,6,7,8,9,10 bekannt sind. Die Unterscheidung hinsichtlich der Strahlenabsorption kann durch Ab- oder Ausschnitte an den Teilobjekten 6,10, deren Dicke, deren Länge, deren Abstand zueinander, deren Material oder deren Form vorgegeben sein.

Das in der FIG 2 gezeigte Röntgendiagnostikgerät unterscheidet sich von dem in der FIG 1 gezeigten Röntgendiagnostikgerät dadurch, daß das Referenzobjekt 2 einen festen Bezug zum Strahlenempfänger 3 hat, mit dem es, beispielsweise über eine Halterung 11, in Verbindung steht.

Eine besonders vorteilhafte Ausgestaltung des Verfahrens ergibt sich, wenn wenigstens drei sich hinsichtlich der Strahlenabsorption unterscheidende Teilobjekte 12,13,14 vorgesehen sind, deren Anordnung zueinander von einer Geraden abweicht. Hierzu können beispielsweise Kugeln vorgesehen sein, deren Strahlenabsorption sich zumindest in Teilbereichen, beispielsweise durch an den Kugeln angebrachte Markierungskörper, unterscheiden und/oder daß deren Projektion auf den Strahlenempfänger 3 hinsichtlich des Ortes zumindest annähernd bekannt ist. In der FIG 5 ist angedeutet, daß ein erstes Teilobjekt 12 beispielsweise als Vollkugel ausgebildet ist. Ein zweites und drittes Teilobjekt 13,14 weisen, beispielsweise als Kugeln ausgebildet, einen Rand auf, der sich hinsichtlich der Strahlenabsorption vom Kern deutlich unterscheidet. Das zweite und dritte Teilobjekt 13,14 unterscheiden sich hinsichtlich der Größe bzw. Dicke des Randes. Als besonderer Vorteil ergibt sich hierdurch, daß bei Verwendung eines solchen Referenzobjektes, das einen festen Bezug zum Untersuchungsobjekt hat, die Position und Lage des Untersuchungsobjektes sowie der Strahlenquelle 1 gegenüber dem Strahlenempfänger 3, die geometrische Anordnung und der zweidimensionale Projektionswinkel ermittelt werden können, so daß eine Tomosyntheseaufnahme des Untersuchungsobjekts rekonstruiert werden kann, ohne daß das Untersuchungsobjekt mit dem Strahlenempfänger 3 oder der Strahlenquelle 1 oder diese untereinander gekoppelt zu sein brauchen. Ein weiterer Vorteil besteht darin, daß die Teilobjekte 12,13,14 des Referenzobjektes unabhängig voneinander in beliebiger relativer Lage am Untersuchungsobjekt angeordnet werden können.

Durch die Auswertung der Abbildungsorte der Teilobjekte auf dem Strahlenempfänger 3 und der bei schräger Projektion auftretenden Verzerrung ihres Strahlenschattenbildes nach Ausmaß und Richtung kann zunächst die Position der Strahlenquelle 1 im Koordinatensystem des Strahlenempfängers 3 bestimmt werden. Ist die Position der Strahlenquelle 1 bekannt, so kann aus dem Abbildungsmaßstab bei bekannter Größe der Teilobjekte auf deren Position geschlossen werden. Mit einer ersten Aufnahme mit einer beliebigen Einstrahlrichtung ist damit eine Anfangslage des Untersuchungsobjektes definiert und spätere Lageänderungen können eindeutig ermittelt werden.

In Abwandlung des Verfahrens kann auch ein Referenzobjekt aus nur zwei hinsichtlich der Strahlenabsorption sich unterscheidenden Teilobjekten, beispielsweise Kugeln, und einem dritten Teilobjekt, das balken- oder stabförmig ausgebildet ist, Anwendung finden. Ein Ausführungsbeispiel eines solchen Referenzobjektes ist in der FIG 6 dargestellt. Es ist gezeigt, daß hierbei ein weiteres erstes Teilobjekt 15 als Kugel ausgebildet ist. An einem weiteren zweiten als Kugel ausgebildeten Teilobjekt 16 ist ein balken- oder stabförmiges Teilobjekt 17 vorgesehen. Hierdurch wird gleichzeitig die Unterscheidbarkeit hergestellt. Die Position der Strahlenquelle 1 kann somit bereits unter Verwendung von nur zwei sich unterscheidenden Teilobjekte 16,17 ermittelt werden. Durch die Auswertung der Projektionsverzerrung der beiden Teilabschnitte des balken- oder stabförmigen Teilobjektes 17 kann eine Rotation des Untersuchungsobjektes um die Verbindungsgerade dieser Teilobjekte 15,16 bestimmt werden.

Werden mehr als drei Teilobjekte bei der Auswertung verwendet, so kann die Genauigkeit der Positions- und Winkelbestimmung verbessert und Redundanz vorgehalten werden, wenn, beispielsweise die Abbildungen einzelner Teilobjekte nicht ausgewertet werden können, weil sich diese beispielsweise mit stark absorbierenden Strukturen des Untersuchungsobjektes überlagern.

## Patentansprüche

1. Verfahren zur Erstellung einer Tomosyntheseaufnahme eines Untersuchungsobjektes mittels eines Röntgendiagnostikgerätes und eines Referenzobjektes (2),
wobei aus den beim Durchstrahlen des aus wenigstens zwei Teilobjekten (5,6,7,8,9,10) bestehenden Referenzobjektes (2) und den beim Durchstrahlen des Untersuchungsobjektes aus unterschiedlichen Richtungen erzeugten und von einem Strahlenempfänger (3) ableitbaren Signalen die Tomosyntheseaufnahme erstellt wird.

2. Verfahren nach Anspruch 1,
wobei das Referenzobjekt (2) wenigstens drei Teilobjekte (7,8,9,10) aufweist, deren Anordnung zueinander von einer Geraden abweicht.

3. Verfahren nach Anspruch 1 oder 2,
wobei die Teilobjekte (7,8,9,10) einen geometrischen Körper bilden.

4. Verfahren nach einem der Ansprüche 1 bis 3,
wobei die Teilobjekte (5,6) kreuzförmig angeordnet sind.

5. Verfahren nach einem der Ansprüche 1 bis 4,
wobei die Teilobjekte (5,6,7,8,9,10) kugel-, scheiben-, balken-, stab- oder zylinderförmig ausgebildet sind.

6. Verfahren nach einem der Ansprüche 1 bis 5,
wobei sich die Teilobjekte (5,6,7,8,9,10) hinsichtlich der Strahlenabsorption unterscheiden.

7. Verfahren nach einem der Ansprüche 1 bis 6,
wobei das Referenzobjekt (2) einen festen Bezug zum Strahlensender, insbesondere zur Strahlenquelle (1), hat, mit dem/der es unmittelbar in Verbindung steht.

8. Verfahren nach einem der Ansprüche 1 bis 6,
wobei das Referenzobjekt (2) einen festen Bezug zum Strahlenempfänger (3) hat, mit dem es unmittelbar in Verbindung steht.

9. Verfahren nach Anspruch 1,
wobei die Teilobjekte zumindest annähernd auf einer Geraden angeordnet sind.

10. Verfahren nach Anspruch 9,
wobei sich die Teilobjekte hinsichtlich der Strahlenabsorption unterscheiden.

11. Vorrichtung zum Durchführen des Verfahrens nach einem der Ansprüche 1 bis 10.
